# EUROPÄISCHE PATENTANMELDUNG

(11) **EP 1 816 119 A1**
(43) Veröffentlichungstag der Anmeldung: **08.08.2007**
(21) Anmeldenummer: 06124461.2
(22) Anmeldetag: 21.11.2006
(51) Int. Cl.: C07C 253/30, C07C 255/17

(54) **Verfahren zur Herstellung von beta-Ketonitrilen und ihren Salzen**

(30) Priorität: 01.12.2005 DE 102005057461
(71) Anmelder: Wacker Chemie AG, 81737 München (DE)
(72) Erfinder: Sorger, Klas, 81371, München (DE); Stohrer, Jürgen, 82049 Pullach (DE)
(74) Vertreter: Fränkel, Robert

(57) **Zusammenfassung**

Verfahren zur Herstellung von β-Ketonitrilen oder deren Salzen durch Umsetzung eines Nitrils mit einem Carbonsäureester in Gegenwart eines Alkali- oder Erdalkalialkoholats, wobei der als Nebenprodukt entstehende Alkohol abdestilliert wird und das so abdestillierte Volumen im wesentlichen volumenmäßig durch Nachdosierung von Nitril laufend ersetzt wird und das Nitril insgesamt im Überschuss bezogen auf den umzusetzenden Carbonsäureester eingesetzt wird.

## Beschreibung

Die Erfindung betrifft ein Verfahren zur Herstellung von β-Ketonitrilen und ihren Salzen. β-Ketonitrile und ihre Salze sind wichtige Synthesebausteine für die Herstellung von pharmazeutischen Wirkstoffen und Pflanzenschutzmitteln.

Aus E. H. Kroeker et al., J. Am. Chem. Soc., 1934, 56, S. 1171 ist die Herstellung von 3-Keto-4-methylvaleronitril durch Umsetzung von Isobuttersäureethylester mit Acetonitril im stöchiometrischen Verhältnis in Gegenwart von Natriumethylat bekannt. Dabei wird Natriumethylat vorgelegt und portionsweise dreimal ein Drittel einer 1:1-Mischung aus Carbonsäureester und Acetonitril zugesetzt, wobei auf den Einsatz eines Überschusses an Nitril verzichtet wird. Jeweils nach Zugabe der Ester-Acetonitril-Mischung wird der als Nebenprodukt gebildete Alkohol abdestilliert. Die erzielbare Ausbeute beträgt dabei lediglich 44 % der Theorie.

Auch aus J. B. Dorsch et al., J. Am. Chem. Soc., 1932, 54, S. 2960 ist ein Verfahren zur Herstellung von α-Benzoalkylcyaniden bekannt, bei dem Ester und Alkoholat in äquimolaren Mengen vorgelegt werden und anschließend ein 25%-iger Überschuss an Nitril zudosiert wird. Nach vollständiger Zudosierung des Nitrils wird der entstehende Alkohol abdestilliert. Nach dem Verfahren sind lediglich Ausbeuten von bis zu 60 % der Theorie möglich.

In EP 220220 B1 ist die Umsetzung von Carbonsäureestern mit einem Überschuss an Acetonitril in Gegenwart der Base Natriummethoxid beschrieben. Durch Einsatz überschüssigen Acetonitrils und Abdestillation des bei der Reaktion gebildeten Alkohols zusammen mit Acetonitril wird das Gleichgewicht auf die Produktseite verschoben, und es kann eine Ausbeute an β-Ketonitril-Natriumsalz von 83 % d. Th. erzielt werden. Dieses Verfahren hat jedoch den Nachteil, dass mit einem sehr großen Überschuss von 13 bis 14 Äquivalenten an Acetonitril bezogen auf den Carbonsäureester gearbeitet wird. Der Großteil an Acetonitril von größer 12 Äquivalenten geht bei der Abdestillation des Alkohols verloren. Zudem führt der hohe Acetonitrilüberschuss zu einem hohen Gesamtvolumen der Reaktionsmischung und damit zu geringen Konzentrationen und schlechten Raum-Zeit-Ausbeuten. Diese Nachteile machen das Verfahren für den industriellen Maßstab unwirtschaftlich.

Aus EP 1316546 A1 ist ein Verfahren bekannt, bei dem Acetonitril mit einem Überschuss an Carbonsäureester in Gegenwart der Base Natriummethoxid zur Reaktion gebracht wird. Durch den Überschuss an Carbonsäureester und Abdestillation von gebildetem Methanol zusammen mit Acetonitril wird das Gleichgewicht der Reaktion auf die Produktseite verschoben, und es können Ausbeuten bis 82 bis 86 % d. Th. bezogen auf den Umsatz an Acetonitril erzielt werden. Das Verfahren hat jedoch den Nachteil, dass ein Großteil des im Überschuss eingesetzten Carbonsäureesters bei der Aufarbeitung verloren geht, was das Verfahren insbesondere für die Umsetzung wertvoller und somit teurer Carbonsäureester-Substrate unwirtschaftlich macht. Zudem wird bei dem Verfahren die gesamte Menge an Carbonsäureester vorgelegt, was zu einem hohen Gesamtvolumen und damit zu niedrigen Konzentrationen und mäßigen Raum-Zeit-Ausbeuten führt. Auch aus diesem Grund ist das Verfahren für den industriellen Maßstab unwirtschaftlich.

Bei den aus EP 220220 B1 und EP 1316546 A1 bekannten Verfahren dient überschüssiges Acetonitril bzw. überschüssiger Carbonsäureester zugleich als Lösemittel mit der Aufgabe die Reaktionsmischung bzw. die sich ausbildende Suspension in gut rührbarer Form zu halten und die Erhöhung der Vikosität (Verbreiung) des Reaktionsgemisches zu vermeiden.

Aus DE 10143858 A1 ist die Umsetzung von Acetonitril mit Oxalsäurediethylester und Natriummethanolat in Gegenwart des Lösemittels tert.-Butylmethylether bekannt unter Anwendung spezieller Reaktionsbedingungen. Bei diesem Verfahren wird Acetonitril nur in einem sehr geringen Überschuss von maximal 15 % bezogen auf den Oxalsäurediester eingesetzt und gebildetes Methanol zur Verschiebung des Gleichgewichts nicht abdestilliert, womit nur eine Ausbeute von maximal 67 % d. Th. erzielt wird. Das Verfahren ist speziell auf Oxalsäurediester als Substrat optimiert und ist wegen der niedrigen erzielbaren Ausbeuten und der durch den Lösungsmitteleinsatz niedrigen Raum-Zeit-Ausbeuten für eine breite industrielle Anwendung zur Herstellung von Ketonitrilen wenig geeignet.

Die aus dem Stand der Technik bekannten Verfahren ermöglichen noch keinen zufriedenstellenden Zugang zur großtechnischen Herstellung von β-Ketonitrilen oder deren Salzen. Dabei sind vor allem hohe chemische Ausbeuten und hohe Konzentrationen der Reaktanden (und somit hohe Raum-Zeit-Leistungen) wünschenswert, wobei vorzugsweise hohe Überschüsse eines Reaktanden vermieden werden sollten.
Es bestand somit die Aufgabe ein alternatives Verfahren zur Herstellung von β-Ketonitrilen oder deren Salzen bereit zu stellen. Insbesondere bestand die Aufgabe, ein für die industrielle Anwendung geeignetes Verfahren bereit zu stellen, das hohe chemische Ausbeuten an β-Ketonitrilen bzw. deren Salzen ermöglicht, mit hohen Raum-Zeit-Ausbeuten arbeitet und große Überschüsse an Acetonitril bzw. Carbonsäureester vermeidet.

Die Aufgabe wurde durch ein neuartiges Verfahren gelöst, bei dem die entstehenden Nebenprodukte aus dem Reaktionssystem entfernt werden und das aus dem Reaktionssystem entfernte Volumen durch Nachdosieren von Nitril ersetzt wird.

Es wurde überraschend gefunden, dass die Herstellung von β-Ketonitrilen bzw. deren Salzen besonders vorteilhaft und wirtschaftlich in hohen Raum-Zeit-Ausbeuten gelingt, wenn während der Umsetzung gebildeter Alkohol abdestilliert und während der Abdestillation von Alkohol Acetonitril nachgesetzt wird, wobei das nachdosierte Nitril in der Gesamtbilanz zu einem eingesetzten Überschuss an Nitril bezogen auf den umgesetzten Ester führt.

Gegenstand der Erfindung ist ein Verfahren zur Herstellung von β-Ketonitrilen der allgemeinen Formel (1a) oder deren Salzen der allgemeinen Formel (1b) 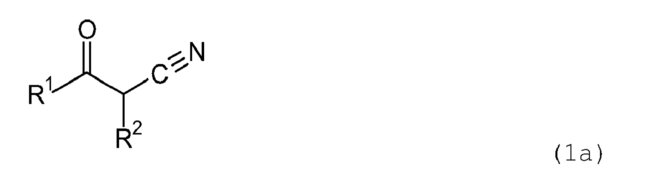 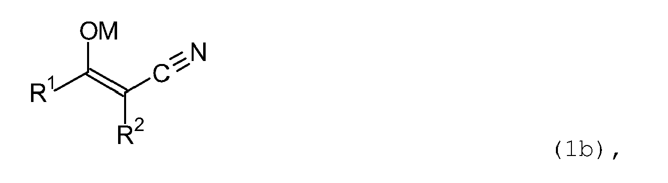 wobei **R¹** für einen linearen oder verzweigten, gesättigten oder ungesättigten, cyclischen oder cyclische Gruppen enthaltenden, nicht-aromatischen, aromatischen oder heteroaromatischen gegebenenfalls mit **Q** substituierten C₁-C₃₀-Kohlenwasserstoff-Rest steht
und **R²** für Wasserstoff oder einen linearen oder verzweigten Alkylrest steht
und **Q** ausgewählt wird aus der Gruppe enthaltend Halogen, Amino, Hydroxy, Cyano, Nitro, Alkoxy, Aryloxy, Alkylthio, Acyl, Silyl, Silyloxy, Aryl, Heteroaryl,
und **M** für Alkali- oder Erdalkaliion steht,
durch Umsetzung eines Nitrils der allgemeinen Formel (2)

R²-CH₂-C≡N (2),

wobei **R²** die oben benannte Bedeutung hat,
mit Carbonsäureester der allgemeinen Formel (3) 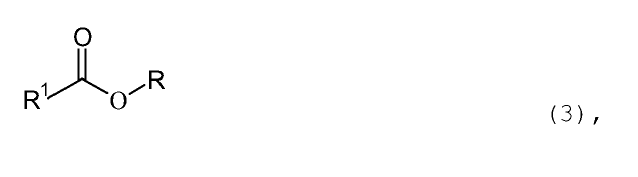 wobei **R¹** die oben benannte Bedeutung hat
und **R** für einen C₁-C₁₀-Alkyl-Rest steht
in Gegenwart eines Alkali- oder Erdalkalialkoholats,
dadurch gekennzeichnet, dass
der als Nebenprodukt entstehende Alkohol R-OH abdestilliert wird, gegebenenfalls in Form eines Azeotrops,
das so abdestillierte Volumen im wesentlichen volumenmäßig durch Nachdosierung von Nitril laufend ersetzt wird und
das Nitril insgesamt im Überschuss bezogen auf den umzusetzenden Carbonsäureester eingesetzt wird.

Kennzeichnendes Merkmal der Erfindung ist, dass die abdestillierte Mengen an Alkohol bzw. Azeotrop durch einen nachdosierten Überschuss an Nitril volumenmäßig kompensiert wird, so dass das Gesamtvolumen der Reaktionsmischung über den Verlauf der Umsetzung im wesentlichen konstant bleibt.

Die wesentliche Auswirkung der erfindungsgemäßen Vorgehensweise ist, dass die stationäre Konzentration des Nitrils während der gesamten Umsetzung nahezu konstant gehalten wird, da das kontinuierlich abdestillierte Volumen, welches im wesentlichen Alkohol oder ein sich typischerweise ausbildendes Azeotrop von Alkohol und Nitril umfasst, volumenmäßig durch das kontinuierliche Nachsetzen von Nitril ersetzt wird. Auf diese Weise verarmt die Reaktionsmischung nicht kontinuierlich an Nitril, es ist aber auch nicht erforderlich mit großen, insbesondere stationären Überschüssen an Nitril zu arbeiten, die zu einer unerwünschten Verdünnung führen und die Raum-Zeit-Ausbeute mindern.

Die alleinige Erhöhung der Nitril-Menge, also das Arbeiten mit einem ständigen ggf. auch sehr hohen Überschuss an Nitril hat sich als negativ herausgestellt und auch zu keiner wesentlichen Gleichgewichtsverschiebung zur Produktseite geführt.

Der Vorteil des erfindungsgemäßen Verfahrens liegt darin, dass der in der Gesamtbilanz insgesamt eingesetzte Überschuss an Nitril deutlich niedriger ausfällt als in den aus dem Stand der Technik bekannten Verfahren und dennoch auf diese Weise deutlich höhere chemische Ausbeuten erzielt werden können.

Mögliche bevorzugte Ausführungsformen für den C₁-C₃₀-Kohlenwasserstoffrest **R¹** sind lineare oder verzweigte, cyclische oder cyclische Gruppen enthaltende, gesättigte oder ungesättigte Alkyl-, Aryl-, Heteroaryl-, Alkenyl- oder Alkinylreste.

In heteroaromatischen C₁-C₃₀-Kohlenwasserstoffresten für R¹ können die Heteroatome bevorzugt ausgewählt werden aus der Gruppe enthaltend Sauerstoff, Schwefel, Stickstoff und Phosphor.

Die C₁-C₃₀-Kohlenwasserstoffreste für **R¹** können gegebenenfalls zusätzlich mit **Q** substituiert sein, wobei **Q** bevorzugt ausgewählt werden kann aus der Gruppe enthaltend F, Cl, Br, J, CN, NO₂, OH, Carbonyl, C₁-C₁₀-Alkoxy-, Aralkyloxy, C₁-C₆-Trialkylsilyl-, C₁-C₆-Trialkylsilyloxy-, NH₂, C₁-C₁₀-Alkylamino-, Di-C₁-C₁₀-alkylamino-, C₁-C₆-Trialkyl-silylamino-, C₁-C₆-Trialkylsilyl-C₁-C₁₀-Alkylamino-, tert.-Butyloxycarbonylamino, Benzyloxycarbonylamino, Aryl-, Aralkyl-, Alkaryl-, Aralkenyl-, Alkenylaryl- oder Heteroaryl-reste, wobei letztere ihrerseits substituiert sein können durch Reste ausgewählt aus der Gruppe F, Cl, Br, J, CN, NH₂, NO₂, C₁-C₁₀-Alkoxyreste, C₁-C₁₀-Alkylaminoreste oder C₁-C₁₀-Alkylreste.

Bevorzugte ungesättigte, aromatische und heteroaromatische Reste für **R¹** werden ausgewählt aus der Gruppe enthaltend Allyl, Vinyl, Furyl, Pyrrolyl, Piperidinyl, Pyrrolidinyl, Quinolinyl, Pyridyl, Piperazinyl, Imidazolyl, Pirimidinyl, Oxazolyl, Isoxazolyl, Morpholinyl, Thiazolyl, Isothiazolyl, Indolyl, Triazinyl, Thienyl, Thiophenyl, Phenyl, Naphthyl, die ihrerseits durch Reste ausgewählt aus der Gruppe enthaltend Methyl, Ethyl, n-Propyl, Isopropyl, n-Butyl, sec.-Butyl, tert.-Butyl, Trifluormethyl, Methoxy, Ethoxy, Phenoxy, Benzyloxy, Amino, N-Methylamino, N,N-Dimethylamino, N-Benzyl, N,N-Dibenzyl, N-Phthalimido, Cyano, Nitro, Hydroxy, Fluor, Chlor, Brom substituiert sein können.

Bevorzugte gesättigte Reste für **R¹** sind lineare oder verzweigte, cyclische oder cyclische Gruppen enthaltende, gegebenenfalls durch Q substituierte, Alkylreste. Besonders bevorzugte Alkylreste für R¹ werden ausgewählt aus der Gruppe enthaltend Methyl, Ethyl, n-Propyl, Isopropyl, n-Butyl, sec.-Butyl, tert.-Butyl, n-Pentyl, n-Hexyl, n-Heptyl, n-Octyl, Cyclopropyl, Cyclobutyl, Cyclopentyl, Cyclohexyl und Cycloheptyl.

Bevorzugte nach dem erfindungsgemäßen Verfahren umzusetzende Carbonsäureester der allgemeinen Formel (3) sind neben aliphatischen, aromatischen oder heteroaromatischen Carbonsäureestern, in denen die Reste R¹ gegebenenfalls durch Q substituiert sind, alpha-, beta- oder gamma-Aminosäureester und alpha-, beta- oder gamma-Hydroxysäureester, bevorzugt in enantiomerenangereicherter oder enantiomerenreiner Form, wobei die Reste R¹ dann einen durch eine Amino- oder Hydroxygruppe substituierten gesättigten oder ungesättigten organischen, gegebenenfalls durch Q substituierten Rest bedeuten. Die Amino- oder Hydroxygruppen in den alpha-, beta- oder gamma-Aminosäureester und alpha-, beta- oder gamma-Hydroxysäureestern können auch substituiert oder geschützt sein.

Besonders bevorzugte Substituenten **Q** sind Fluor, Chlor, Brom, Nitro, Cyano, Carbonyl, Hydroxy, Amino, N-Methylamino, N,N-Dimethylamino, N-Benzyl, N,N-Dibenzyl, N-Acetylamino, N-Acetyl-N-methylamino, tert.-Butyloxycarbonylamino, N-Benzyloxycarbonylamino, Methoxy, Ethoxy, tert.-Butyloxy, Phenoxy, Benzyloxy, Acetyl, Propionyl, Pivalinoyl, Phenyl, Naphthyl, Benzyl, Furyl, Piperidinyl, Pyrrolidinyl, Quinolinyl, Pyridyl, Piperazinyl, Imidazolyl, Pirimidinyl, Oxazolyl, Isoxazolyl, Morpholinyl, Thiazolyl, Isothiazolyl, Indolyl, Triazinyl, Thienyl oder Thiophenyl, die wiederum durch Reste ausgewählt aus der Gruppe enthaltend Methyl, Ethyl, n-Propyl, Isopropyl, Butyl, sec.-Butyl, tert.-Butyl, Cyclopropyl, Cyclobutyl, Cyclopentyl, Cyclohexyl, Allyl, Vinyl, Phenyl, Furyl, Piperidinyl, Pyrrolidinyl, Quinolinyl, Pyridyl, Piperazinyl, Imidazolyl, Pirimidinyl, Oxazolyl, Isoxazolyl, Morpholinyl, Thiazolyl, Isothiazolyl, Indolyl, Triazinyl, Thienyl, Thiophenyl, Fluor, Chlor, Brom, Nitro, Cyano, Amino, Hydroxy, Methoxy, Ethoxy, Phenoxy, Trimethylsilyl, Triethylsilyl, Acetyl, Propionyl, Amino, N,N-Dimethylamino, N-Benzyl, N-Acetylamino, N-Acetyl-N-methylamino oder N-Benzyloxycarbonylamino substituiert sein können sowie Acetyl-, Amino-, N-Methylamino-, N,N-Dimethylamino-, N-Benzyl-, N-Acetylamino-, N-Acetyl-N-methylamino-, N-Benzyloxycarbonylamino-, Nitro-, Methyl-, Ethyl-, Propyl-, Isopropyl-, Butyl-, sec.-Butyl-, tert.-Butyl-, Methoxy-, Ethoxy-, Phenoxy-, Acetoxy-, Benzyloxy-, Trimethylsilyl-, Trimethylsilyloxy, Triethylsilyloxy-, Fluor-, Chlor-, Brom-, Jod- und Cyanophenyl oder -naphthyl.

Bevorzugte Reste für **R²** sind Wasserstoff und C₁-C₁₀-Alkylreste, insbesondere Methyl, Ethyl, n-Propyl, Isopropyl, n-Butyl, sec-Butyl, tert-Butyl, hierbei insbesondere Methyl, Ethyl, n-Propyl und Isopropyl.

Besonders bevorzugte Nitrile der allgemeinen Formel (2) werden ausgewählt aus der Gruppe enthaltend Acetonitril, Propionitril und Butyronitril, insbesondere Acetonitril.

In den Estern der allgemeinen Formel (2) werden die C₁-C₁₀-Alkylreste **R** bevorzugt ausgewählt aus der Gruppe enthaltend Methyl, Ethyl, n-Propyl, Isopropyl, n-Butyl, sec.-Butyl, tert.-Butyl, insbesondere Methyl, Ethyl, n-Propyl und Isopropyl.

Als Alkoholate sind Alkali und Erdalkalialkoholate geeignet. Bevorzugt werden Alkalialkoholate eingesetzt, insbesondere Natrium- und Kaliummethylat, -ethylat, -propylat, -butylat, -pentylat, besonders bevorzugt Natriummethylat und -ethylat. Das Alkoholat wird in fester Form oder als Lösung in Alkohol oder anderen geeigneten Lösemitteln eingesetzt, bevorzugt in fester Form, was sich vorteilhaft auf die Raum-Zeit-Ausbeuten auswirkt.

Die Alkoholate werden im Verhältnis 0.5 bis 2, bevorzugt 0.7 bis 1.5, besonders bevorzugt 0.9 bis 1.1 : 1, bezogen auf den Carbonsäureester eingesetzt. Insbesondere bevorzugt ist der stöchiometrische (äquimolare) Einsatz von Alkoholat und Carbonsäureester.

Das Nitril, insbesondere Acetonitril wird insgesamt im Überschuss bezogen auf den Carbonsäureester eingesetzt, wobei das Verhältnis von Nitril zu umzusetzendem Carbonsäureester 2 bis 10 : 1, bevorzugt 3 bis 7 : 1, besonders bevorzugt 4 bis 6 : 1 beträgt.

In einer möglichen Ausführungsform des erfindungsgemäßen Verfahrens werden zuerst 0.3 bis 2, bevorzugt 0.5 bis 1.5, besonders bevorzugt 0.9 bis 1.1 Äquivalente Nitril, insbesondere Acetonitril bezogen auf Carbonsäureester zusammen mit Carbonsäureester und Alkoholat vorgelegt. Auf diese Weise wird eine sehr hohe Konzentration der Mischung erreicht. Die Reihenfolge des Zusammenmischens von Nitril , Carbonsäureester und Alkoholat kann beliebig erfolgen, bevorzugt wird Alkoholat vorgelegt, dann Nitril und Carbonsäureester zugegeben. Es ist auch möglich Alkoholat und Carbonsäureester vorzulegen und dann Nitril, gegebenenfalls bei erhöhter Temperatur, zuzusetzen.

Die Umsetzung erfolgt bei erhöhter Temperatur zwischen 50 und 150 °C, bevorzugt bei 70 bis 120 °C.

Mit fortschreitender Umsetzung wird Alkohol gebildet. Zur Verschiebung des Gleichgewichts wird der im Rahmen der Umsetzung gebildete Alkohol oder das sich in Abhängigkeit von der Wahl des Nitrils, insbesondere bei der Verwendung von Acetonitril sich regelmäßig ausbildende azeotrope Gemisch aus Alkohol und Nitril oder, im Falle niedrig siedender Carbonsäureester, gegebenenfalls das azeotrope Gemisch aus Alkohol, Nitril und Carbonsäureester fortlaufend aus dem Reaktionsgemisch abdestilliert. Da insbesondere bei Einsatz von Acetonitril der entstehende Alkohol regelmäßig zusammen mit dem Acetonitril als Azeotrop abdestilliert wird, setzt man während der Umsetzung laufend Acetonitril nach, entweder kontinuierlich oder portionsweise. Das gleiche gilt beim Einsatz eines anderen Nitrils.

Dabei entspricht das Volumen an abdestilliertem Alkohol bzw. des Azeotrops, insbesondere des Alkohol-Acetonitril-Gemisches, näherungsweise dem Volumen an nachgesetztem Nitril. Auf diese Weise findet die Umsetzung bei höchstmöglicher Konzentration statt, was zu sehr hohen Raum-Zeit-Ausbeuten führt.

Das Nitril, insbesondere Acetonitril wird in der Regel im Verhältnis 0.5 bis 9 : 1, bevorzugt 1 bis 6 : 1, besonders bevorzugt 2 bis 5 : 1 bezogen auf Carbonsäureester nachgesetzt.

In dem erfindungsgemäßen Verfahren wird so insgesamt in der Regel ein 2 bis 10-facher, vorzugsweise 3 bis 7-facher, insbesondere 4 bis 6-facher Überschuss an Nitril, insbesondere an Acetonitril eingesetzt.
Entgegen den aus dem Stand der Technik bekannten Verfahren wurde überraschenderweise gefunden, dass durch die Nachdosierung des Nitrils während der Abdestillation des entstehenden Alkohols bzw. dessen Azeotrop mit dem Nitril auf einen hohen, insbesondere stationären Überschuss an Acetonitril (wie zum Beispiel in dem aus EP220022 B1 bekannten Verfahren von 12 bis 13 Äquivalenten) bezogen auf Carbonsäureester verzichtet werden kann.

Insbesondere können nach dem erfindungsgemäßen Verfahren bereits hohe chemische Ausbeuten von > 85-90 % der Theorie bei nahezu vollständigem Umsatz bezogen auf den Carbonsäureester erreicht werden. Hierfür werden insgesamt überraschenderweise in der Regel nur 3 bis 6 Äquivalente an Nitril, wobei davon vorzugsweise nur 2 bis 5 Äquivalente während der Reaktion nachgesetzt werden müssen, eingesetzt. Die laufende Zudosierung von Nitril während der Abdestillation von Alkohol hat somit überraschenderweise einen besonders vorteilhaften Einfluss auf die Umsetzung.

In einer besonders bevorzugten Ausführungsform des erfindungsgemäßen Verfahrens wird die gesamte umzusetzende Menge an Carbonsäureester und Alkoholat, bevorzugt im stöchiometrischen (äquimolaren) Verhältnis, mit einem Teil des insgesamt eingesetzten Nitrils vorgelegt, was sich vorteilhaft auf die Reaktionszeit, die Raum-Zeit-Ausbeute und den Verlauf der gesamten Umsetzung auswirkt.

Wird beispielsweise eine Suspension aus einem Äquivalent Natriummethylat, einem Äquivalent Cyclopropancarbonsäuremethylester und einem Äquivalent Acetonitril 1 h auf 90 °C erwärmt, so bildet sich trotz der hohen Konzentration der Reaktanden und Abwesenheit von zusätzlichem Lösemittel überraschenderweise eine klare Lösung, aus der unter Nachsetzen von Acetonitril technisch einfach und ohne Verbreiung Methanol bzw. ein Gemisch aus Methanol und Acetonitril abdestilliert und das Gleichgewicht der Umsetzung auf die Produktseite verschoben werden kann (vgl. Beispiel 1a). Überraschend ist dabei insbesondere die Tatsache, dass sich bei Temperaturen von mehr als 70 °C nach kurzer Reaktionszeit eine klare homogene, leicht bewegliche Lösung bildet, ohne dass Lösemittel wie z.B. polar aprotische Lösemittel, insbesondere überschüssiges Acetonitril, oder Ether, wie z. B. Tetrahydrofuran oder Methyl-tert.-butylether zugesetzt werden müssen.

Ein hoher Überschuss an Acetonitril oder Carbonsäureester, der in den vorbekannten Verfahren als Lösemittel zur Vermeidung der Verbreiung dient, ist damit nicht erforderlich. Wird dagegen wie in E. H. Kroeker et al., J. Am. Chem. Soc., 1934, 56, S. 1171 beschrieben überschüssiges Alkoholat bezogen auf stöchiometrische Mengen an Carbonsäureester und Acetonitril vorgelegt, so bildet sich beim Erwärmen eine breiige, schwer rührbare Suspension, die zur Krustenbildung an den Gefäßwänden und Verklebung neigt, was sich nachteilig auf Reaktionszeiten, Ausbeuten und Reinheiten der hergestellten Produkte auswirkt und insbesondere bei der großtechnischen Umsetzung zu Problemen führt.

Wird hingegen nach dem erfindungsgemäßen Verfahren eine stöchiometrische Mischung aus Alkoholat, Nitril und Ester ohne Lösemittel erwärmt und während der Abdestillation von Alkohol-Nitril-Gemisch laufend Nitril nachgesetzt, dann erfolgt nahezu vollständiger Umsatz bezogen auf Ester in überraschenderweise sehr kurzen Reaktionzeiten. Wird beispielsweise eine Mischung aus einem Äquivalent Natriummethylat, einem Äquivalent Cyclopropancarbonsäuremethylester mit insgesamt 4.5 Äquivalenten Acetonitril, von denen zunächst nur ein Äquivalent zusammen mit den anderen Reaktanden vorgelegt wurde, auf 90 °C erwärmt, anschließend das sich bildende Methanol-Acetonitril-Azeotrop abdestilliert und eine dem Volumen nach im wesentlichen identische Menge an Acetonitril kontinuierlich nachgesetzt, so ist der eingesetzte Cyclopropancarbonsäuremethylester bereits nach insgesamt nur 3.5 h vollständig umgesetzt. Im Gegensatz hierzu wird nach dem aus E. H. Kroeker et al., J. Am. Chem. Soc., 1934, 56, S. 1171 bekannten Verfahren bei Einsatz stöchiometrischer Mengen an Acetonitril, Natriumethoxid und Isobuttersäureethylester nach 9 h bei 115 bis 120 °C nur 44 % Ausbeute des Ketonitrils erhalten.

Überraschend ist zudem, dass bei Durchführung der Umsetzung nach dem erfindungsgemäßen Verfahren nur sehr geringe Verluste durch Eigenkondensation von Carbonsäureester oder Nitril, insbesondere Acetonitril entstehen. Wird dagegen auf die Zugabe des Nitrils zur Mischung aus Ester und Alkoholat verzichtet, so beobachtet man insbesondere für Ester aliphatischer Carbonsäuren beim Erwärmen der Mischung aus Ester und Alkoholat die unerwünschte Bildung von Kondensationsprodukten des Esters bedingt durch basenkatalysierte Eigenkondensation. Derartige Kondensationsprodukte können nach dem erfindungsgemäßen Verfahren überraschenderweise fast vollständig vermieden werden.

Durch den dem erfindungsgemäßen Verfahren immanenten geringen stationären Überschuss an Acetonitril in der Reaktionsmischung werden auch unerwünschte Eigenkondensationsprodukte des Acetonitrils nur mit minimalem Anteil gebildet. Die Zudosierung von Acetonitril zu einer klaren stöchiometrischen Mischung aus Natriummethoxid, Carbonsäureester und Acetonitril bei gleichzeitigem Abdestillieren von Methanol-Acetonitrilgemisch wirkt sich dabei besonders vorteilhaft auf die Minimierung von Eigenkondensationsprodukten des Acetonitrils und Polymerisation von Acetonitril aus.

Wird dagegen wie in EP 220220 beschrieben eine Mischung aus einem Teil Capronsäuremethylester und 13 Teilen Acetonitril zum Rückfluss erhitzt, ein Teil der methanolischen Natriummethanolat-Lösung zudosiert und dabei eine Mischung aus Methanol und Acetonitril abdestilliert, so bilden sich erheblich mehr Anteile an unerwünschten Nebenprodukten, die aus der Selbstaddition bzw. -kondensation des im hohen Überschuss vorhandenen Acetonitrils in Gegenwart der Base resultieren.

In einer besonders bevorzugten Ausführungsform des erfindungsgemäßen Verfahrens wird eine stöchiometrische Mischung aus Alkoholat, Nitril und Carbonsäureester unter Verzicht auf zusätzliches Lösemittel vorgelegt. Die Mischung wird erwärmt und mit einsetzender Abdestillation von Alkohol bzw. Alkohol-Nitril-Gemisch wird Nitril kontinuierlich solange nachdosiert, bis nahezu vollständiger Umsatz bezogen auf Carbonsäureester erreicht ist. Anschließend werden Reste an Alkohol und Nitril soweit wie möglich abdestilliert, gegebenenfalls nach Zugabe eines inerten Lösemittels. Das sich zunächst bildende Ketonitrilsalz wird abschließend abfiltriert oder alternativ die Mischung zur Freisetzung des Ketonitrils wässrig aufgearbeitet. Auf diese Weise können besonders hohe Produktausbeuten und -reinheiten in kurzen Reaktionszeiten von 3 bis 6 h bei höchstmöglicher Konzentration und besonders hohen Raum-Zeit-Ausbeuten und somit insgesamt sehr wirtschaftlicher Reaktionsführung erzielt werden.

Die erfindungsgemäße Umsetzung des Carbonsäureesters mit dem Nitril in Gegenwart eines Alkoholats kann auch in Gegenwart eines inerten Lösemittels durchgeführt werden.

Als inerte Lösemittel sind Ether wie beispielsweise tert.-Butylmethylether, Dibutylether, Dimethoxyethan oder Diethoxyethan, hochsiedende Glykole wie beispielsweise Polyethylenglykole, Alkohole wie beispielweise Isopropanol, n-Butanol, 2-Butanol oder tert.-Butanol, Kohlenwasserstoffe wie beispielsweise Toluol, Xylol oder Mesitylen oder polar aprotische Lösemittel wie beispielsweise N,N-Dimethylformamid, Dimethylsulfoxid oder N-Methylpyrrolidon geeignet, insbesondere Dibutylether, Dimethoxyethan, Diethoxyethan, Toluol, N,N-Dimethylformamid und Dimethylsulfoxid, bevorzugt Toluol.

In einer besonders bevorzugten Ausführungsform des erfindungsgemäßen Verfahrens wird die Umsetzung jedoch ohne Zusatz von zusätzlichen inerten Lösemitteln durchgeführt, was sich vorteilhaft auf die Raum-Zeit-Ausbeute auswirkt.

Gegen Ende der Umsetzung kann es zur Ausfällung von gebildetem Ketonitrilsalz der allgemeinen Formel (1b) kommen. Um die Ausfällung zu vervollständigen ist es möglich, restliches Nitril, gegebenenfalls zusammen mit Resten an Alkohol oder nicht umgesetztem Ester, abzudestillieren. Das Ketonitrilsalz kann anschließend durch Filtration isoliert werden. Gegebenenfalls kann die Filtration in Gegenwart eines inerten Lösemittels durchgeführt werden, insbesondere wenn die Mischung bei Temperaturen unterhalb der Reaktionstemperatur, insbesondere zwischen 10 und 80 °C, filtriert wird.

Als inerte Lösemittel sind die oben genannten Lösemittel geeignet, insbesondere Toluol, Dibutylether, tert.-Butylmethylether, Dimethoxyethan oder Diethoxyethan.

Die nach dem erfindungsgemäßen Verfahren hergestellten Ketonitrilsalze sind überraschenderweise von sehr hoher Reinheit und können in den meisten Fällen direkt weiterverarbeitet werden. Gegebenenfalls können die Ketonitrilsalze auch weiter gereinigt werden, beispielsweise durch Waschung, Umkristallisation oder Extraktion. Aus den gereinigten Ketonitrilsalzen lassen sich die Ketonitrile in besonders reiner Form freisetzen.

Zur direkten Freisetzung von Ketonitril aus hergestelltem Ketonitrilsalz wird die Mischung nach erfolgter Umsetzung mit Wasser oder wässriger Säure hydrolysiert. Die Hydrolyse ist auch bei deutlich höheren Temperaturen als Raumtemperatur möglich, ohne dass merklich Ausbeuteverluste auftreten. Die Hydrolyse erfolgt bei Temperaturen zwischen 0 und 100 °C, bevorzugt 30 bis 80 °C. Zur Hydrolyse wird Wasser oder wässrige Säure wie beispielsweise Salz- oder Schwefelsäure in die Reaktionsmischung eingebracht, bis sich Feststoffe vollständig lösen. Alternativ ist es auch möglich, die Reaktionsmischung auf Wasser oder wässrige Säure zu dosieren. Wird zur Hydrolyse Wasser eingesetzt, dann löst sich Ketonitrilsalz in Form seines Enolats der allgemeinen Formel (1b) in der wässrigen alkalischen Phase. Gegebenenfalls kann die wässrige Phase mit organischem, mit Wasser nicht mischbarem Lösemittel extrahiert werden. Die wässrige Phase wird dann mit einer Säure auf pH 0 bis 8, bevorzugt 2 bis 5 angesäuert, wobei Ketonitril der allgemeinen Formel (1a) freigesetzt wird. Wird zur Hydrolyse direkt wässrige Säure verwendet, dann wird Ketonitril unmittelbar freigesetzt. Das freigesetzte Ketonitril wird abschließend mit organischem, mit Wasser nicht mischbarem Lösemittel extrahiert und gegebenenfalls gereinigt, wie beispielsweise durch Filtration, Destillation, Kristallisation oder Extraktion.

Es ist auch möglich, die Aufarbeitung und Freisetzung von Ketonitril durch Zugabe einer nicht-wässrigen Säure, wie beispielsweise Ameisensäure oder Essigsäure, durchzuführen oder die Reaktionsmischung auf die nicht-wässrige Säure zu dosieren. Freigesetztes Ketonitril kann anschließend direkt durch Destillation, Filtration oder gegebenenfalls nach Zugabe von Wasser oder einer wässrigen Säure durch Extraktion mit organischem, mit Wasser nicht mischbarem Lösemittel isoliert werden.

Bevorzugt wird das Ketonitrilsalz durch Filtration isoliert oder Ketonitril durch wässrige Aufarbeitung freigesetzt.

Der Druckbereich der Reaktion ist unkritisch und kann innerhalb weiter Grenzen variiert werden. Der Druck beträgt üblicherweise 0.01 bis 20 bar, bevorzugt wird die Reaktion unter Normaldruck (Atmosphärendruck) durchgeführt.

Die Reaktion wird bevorzugt unter Inertisierung mit einem inerten Schutzgas, insbesondere Stickstoff oder Argon durchgeführt. Die Reaktion kann kontinuierlich oder diskontinuierlich durchgeführt werden.

Alle vorstehenden Symbole der vorstehenden Formeln weisen ihre Bedeutungen jeweils unabhängig voneinander auf.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C. Die Beispiele dienen der weiteren Veranschaulichung des erfindungsgemäßen Verfahrens und sind in keiner Weise als Einschränkung zu betrachten.

### Beispiel 1a:

### Herstellung von 3-Cyclopropyl-3-ketopropionitril-Natriumsalz (Aufarbeitung durch Filtration)

Bei Raumtemperatur wurden in einem Vierhalskolben mit Innenthermometer, Tropftrichter, KPG-Rührer und aufgesetzter Destillationsapparatur mit Trennkolonne unter Stickstoff-Schutzgas 21.6 g festes Natriummethoxid (0.4 mol) vorgelegt und 16.4 g Acetonitril (0.4 mol) und 40.0 g Cyclopropancarbonsäuremethylester (0.4 mol) zudosiert. Die Suspension wurde auf 85 °C erwärmt und 1 h gerührt, wobei sich eine klare Mischung bildete. Anschließend wurde eine Mischung aus Methanol und Acetonitril bei einer anfänglichen Kopftemperatur von 63 °C abdestilliert. Mit Beginn der Abdestillation wurde in die Reaktionsmischung Acetonitril nachdosiert (insgesamt 74 g, 1.8 mol), wobei das Volumen an nachgesetztem Acetonitril dem abdestillierten Methanol-Acetonitril-Gemisch entsprach. Im Laufe der Abdestillation wurde die Innentemperatur der Reaktionsmischung auf 90 bis 95 °C erhöht. Gegen Ende der Abdestillation von MeOH/Acetonitril bildete sich eine Suspension. Zuletzt wurde fast reines Acetonitril bei einer Kopftemperatur von 80 °C abdestilliert (Zusammensetzung des gesamten Destillats in Gewichts-% nach GC: 72.4 % Acetonitril, 26 % Methanol, 1.6 % Cyclopropancarbonsäuremethylester). Umsatzkontrolle (NMR, GC) zeigte, dass der Ester bis auf 2 Mol-% umgesetzt war. Anschließend wurden 40 ml Toluol zum Reaktionsgemisch zugefügt, und restliches Acetonitril wurde abdestilliert (kann zurückgewonnen werden). Die Mischung wurde auf 70 °C abgekühlt und über eine Glasfritte filtriert. Der Filterkuchen wurde zweimal mit je 20 ml Aceton gewaschen und im Vakuum getrocknet. Es wurden 45.1 g 3-Cyclopropyl-3-ketopropionitril-Natriumsalz von leicht gelblicher Farbe erhalten, was einer Ausbeute von 86 % d. Th. entspricht (chem. Reinheit: > 95 %).

### Beispiel 1b:

### Herstellung von 3-Cyclopropyl-3-ketopropionitril (wässrige Aufarbeitung)

Zunächst analoge Durchführung wie im Beispiel 1a. Die toluolhaltige Suspension wurde auf 50 °C abgekühlt. Unter Kühlung wurden 40 ml Wasser zudosiert, wobei sich eine klare Lösung bildete. Anschließend wurde mit 20 %-iger Salzsäure auf pH 4 gestellt. Nach Phasentrennung wurde die wässrige Phase zweimal mit je 20 ml Methylenchlorid extrahiert. Die vereinigten organischen Phasen wurden mit 20 ml 8 %-iger NaHCO₃-Lsg. gewaschen. Nach Abdestillieren von Lösemittel wurde 3-Cyclopropyl-3-ketopropionitril von leicht bräunlicher Farbe in einer Ausbeute von 36.2 g erhalten (83 % d. Th., chem. Reinheit: 98 %).

### Beispiel 2a:

### Herstellung von 4,4-Dimethyl-3-ketovaleronitril-Natriumsalz (Aufarbeitung durch Filtration)

In analoger Durchführung zum Beispiel 1a unter Einsatz von 10 g Pivalinsäuremethylester (0.086 mol), 4.65 g NaOMe (0.086 mol) und 17.7 g Acetonitril (0.43 mol), von denen 3.5 g (0.086 mol) vorgelegt wurden, wurden nach Filtration, Waschung und Trocknung 12.7 g des Natriumsalzes von 4,4-Dimethyl-3-ketovaleronitril (93 % d. Th.) in einer Reinheit von 97 % erhalten.

### Beispiel 2b:

### Herstellung von 4,4-Dimethyl-3-ketovaleronitril (wässrige Aufarbeitung)

Analoge Durchführung wie im Beispiel 1a bei Einsatz der in Beispiel 2a verwendeten Mengen lieferten 9.8 g 4,4-Dimethyl-3-ketovaleronitril (91 % d. Th.) in einer Reinheit von 98.2 % (GC).

### Beispiel 3:

### Herstellung von 3-Ketocapronitril (wässrige Aufarbeitung)

In analoger Durchführung zu Beispiel 1b wurden 10 g Buttersäuremethylester (0.098 mol), 5.28 g NaOMe (0.098 mol) und 4.02 g Acetonitril (0.098 mol) vorgelegt, und die Mischung wurde auf 85 °C erwärmt. Nach 2 h wurde mit der Abdestillation einer Mischung aus MeOH und Acetonitril begonnen, und mit Beginn der Abdestillation wurden 16.1 g Acetonitril (0.39 mol) kontinuierlich nachdosiert. Nach weiteren 2.5 h Abdestillation von MeOH/Acetonitril und Nachdosieren von Acetonitril wurden 20 ml Toluol zugesetzt, und restliches MeOH/Acetonitril wurde abdestilliert. Nach Abkühlung auf 60 °C fügte man 10 %-ige Salzsäure bis zu einem pH-Wert von 3 zu. Nach Phasentrennung wurde die wässrige Phase zweimal mit je 20 ml Methylenchlorid extrahiert. Die organische Phase wurde einmal mit 15 ml 5 %-iger NaHCO₃-Lsg. gewaschen. Nach Abdestillieren der Lösemittel Toluol und Methylenchlorid wurde 3-Ketocapronitril in Form einer fast farblosen Flüssigkeit in einer Ausbeute von 8.5 g bezogen auf umgesetzten Buttersäureester erhalten (89 % d. Th., chem. Reinheit: 98.6 %). Nach GC wurden 12 Mol-% Buttersäuremethylester zusammen mit abdestilliertem MeOH und Aceteonitril verloren.

### Beispiel 4:

### Herstellung von 3-Phenylpropionitril (wässrige Aufarbeitung)

In analoger Durchführung zum Beispiel 1b wurden 10 g Benzoesäuremethylester (0.074 mol), 4.0 g NaOMe (0.074 mol) und 3.01 g Acetonitril (0.074 mol) vorgelegt, und die Mischung wurde auf 90 °C erwärmt. Nach 1.5 h bildete sich eine klare Lösung, und es wurde mit der Abdestillation einer Mischung aus MeOH und Acetonitril begonnen. Mit Beginn der Abdestillation wurden 12.4 g Acetonitril (0.29 mol) kontinuierlich nachdosiert. Nach weiteren 2.5 h Abdestillation von MeOH/Acetonitril und Nachdosieren von Acetonitril wurden 20 ml Toluol zugesetzt, und restliches MeOH/Acetonitril wurde abdestilliert. Nach Abkühlung auf 40 °C fügte man 10 %-ige Salzsäure bis zu einem pH-Wert von 3 zu, wobei sich gebildete Feststoffe auflösten. Nach Phasentrennung wurde die wässrige Phase zweimal mit je 20 ml Methylenchlorid extrahiert. Die organische Phase wurde einmal mit 10 ml 8 %-iger NaHCO₃-Lsg. gewaschen. Nach Abdestillieren der Lösemittel Toluol und Methylenchlorid wurde 3-Phenylpropionitril in Form eines fast farblosen Feststoffs in einer Ausbeute von 10.1 g erhalten (92 % d. Th., chem. Reinheit: 97 %). Nach Umkristallisation aus Ethanol wurde farbloser Feststoff mit einem Smp. von 82 °C erhalten.

## Patentansprüche

1. Verfahren zur Herstellung von β-Ketonitrilen der allgemeinen Formel (1a) oder deren Salzen der allgemeinen Formel (1b) 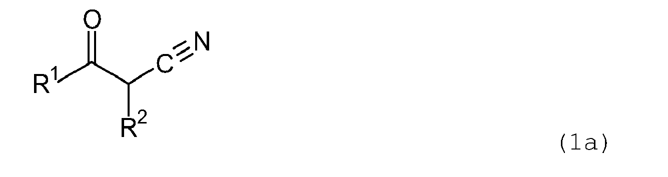 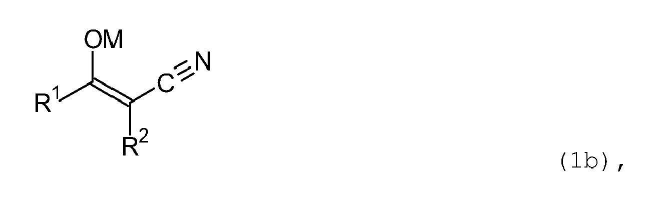 wobei **R¹** für einen linearen oder verzweigten, gesättigten oder ungesättigten, cyclischen oder cyclische Gruppen enthaltenden, nicht-aromatischen, aromatischen oder heteroaromatischen gegebenenfalls mit **Q** substituierten C₁-C₃₀-Kohlenwasserstoff-Rest steht
und **R²** für Wasserstoff oder einen linearen oder verzweigten Alkylrest steht
und **Q** ausgewählt wird aus der Gruppe enthaltend Halogen, Amino, Hydroxy, Cyano, Nitro, Alkoxy, Aryloxy, Alkylthio, Acyl, Silyl, Silyloxy, Aryl, Heteroaryl,
und **M** für Alkali- oder Erdalkaliion steht,
durch Umsetzung eines Nitrils der allgemeinen Formel (2)
R²-CH₂-C≡N (2),
wobei **R²** die oben benannte Bedeutung hat,
mit Carbonsäureester der allgemeinen Formel (3) 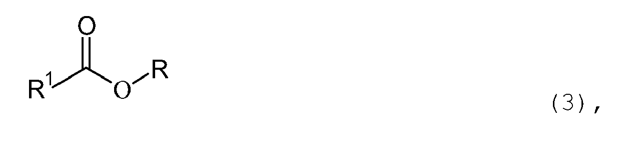 wobei **R¹** die oben benannte Bedeutung hat
und **R** für einen C₁-C₁₀-Alkyl-Rest steht
in Gegenwart eines Alkali- oder Erdalkalialkoholats,
**dadurch gekennzeichnet, dass**
der als Nebenprodukt entstehende Alkohol R-OH abdestilliert wird, gegebenenfalls in Form eines Azeotrops,
das so abdestillierte Volumen im wesentlichen volumenmäßig durch Nachdosierung von Nitril laufend ersetzt wird und
das Nitril insgesamt im Überschuss bezogen auf den umzusetzenden Carbonsäureester eingesetzt wird.

2. Verfahren nach Anspruch 1, **dadurch gekennzeichnet, dass** der Carbonsäureester, das Nitril und das Alkali- oder Erdalkalialkoholat zu Beginn der Umsetzung in im wesentlichen äquimolaren Mengen vorgelegt werden.

3. Verfahren nach Anspruch 1 oder 2, **dadurch gekennzeichnet, dass** der gesamte eingesetzte Überschuss an Nitril bezogen auf den umzusetzenden Carbonsäureester 4 bis 6 : 1 beträgt.

4. Verfahren nach einem oder mehreren der Ansprüche 1 bis 3, **dadurch gekennzeichnet, dass** die laufende Nachdosierung des Nitrils kontinuierlich oder portionsweise erfolgt.

5. Verfahren nach einem oder mehreren der Ansprüche 1 bis 4, **dadurch gekennzeichnet, dass** als Alkalialkoholat Natriummethylat oder Natriumethylat eingesetzt werden.

6. Verfahren nach einem oder mehreren der Ansprüche 1 bis 5, **dadurch gekennzeichnet, dass** das Nitril ausgewählt wird aus der Gruppe enthaltend Acetonitril, Propionitril und Butyronitril.

7. Verfahren nach einem oder mehreren der Ansprüche 1 bis 5, **dadurch gekennzeichnet, dass** die Carbonsäureester ausgewählt werden aus der Gruppe enthaltend gegebenenfalls durch Q substituierte und gegebenenfalls enantiomerenangereicherte oder enantiomerenreine alpha-, beta- oder gamma-Aminosäureester oder alpha-, beta- oder gamma-Hydroxysäureester.

8. Verfahren nach einem oder mehreren der Ansprüche 1 bis 7, **dadurch gekennzeichnet, dass** die Umsetzung bei 70 bis 120 °C erfolgt.

9. Verfahren nach einem oder mehreren der Ansprüche 1 bis 8, **dadurch gekennzeichnet, dass** die Umsetzung in Gegenwart eines inerten Lösemittels durchgeführt wird.

10. Verfahren nach einem oder mehreren der Ansprüche 1 bis 9, **dadurch gekennzeichnet, dass** die Umsetzung ohne Zusatz von zusätzlichen inerten Lösemitteln durchgeführt wird.
